**Europäisches Patentamt**

**European Patent Office**

**Office européen des brevets**

(19)

(11) Veröffentlichungsnummer: **0 425 678 A1**

(12) **EUROPÄISCHE PATENTANMELDUNG**
veröffentlicht nach Art. 158 Abs. 3
EPÜ

(21) Anmeldenummer: **90902574.4**

(22) Anmeldetag: **04.10.89**

(86) Internationale Anmeldenummer:
**PCT/SU89/00260**

(87) Internationale Veröffentlichungsnummer:
**WO 90/14114 (29.11.90 90/27)**

(51) Int. Cl.⁵: **A61M 5/00**

(30) Priorität: **16.05.89 SU 4684318**

(43) Veröffentlichungstag der Anmeldung:
**08.05.91 Patentblatt 91/19**

(84) Benannte Vertragsstaaten:
**BE CH DE FR GB IT LI SE**

(71) Anmelder: **POLTAVSKY MEDITSINSKY
STOMATOLOGICHESKY INSTITUT
ul. Shevchenko, 23,
Poltava 314000(SU)**

(72) Erfinder: **MAZURIK, Sergei Mikhailovich
ul. Lenina, 92-57
Poltava, 314022(SU)**
Erfinder: **SOKOLOV, Andrei Nikolaevich
ul. 60 let SSSR, 3-20
Poltava, 314022(SU)**

(74) Vertreter: **Nix, Frank Arnold, Dr.
Kröckelbergstrasse 15
W-6200 Wiesbaden(DE)**

(54) **EINMALSPRITZE FÜR INJEKTIONEN.**

(57) Die Erfindung bezieht sich auf die Medizintechnik.

Die Einmalspritze für Injektionen hat einen Zylinder (1), einen Kolben (6) mit Kolbenstange (4) und einen Ansatz zum Aufsetzen der Nadel am vorderen Stirnende des Zylinders (1). Die Kolbenstange (4) hat auf ihrer Länge zwei Abschnitte (7, 8) verschiedenen Querschnitts: Der Abschnitt (7) hat die Form eines Stabes und der Abschnitt (8) ist im Querschnitt kreuzförmig ausgebildet und durch zwei zueinander rechtwinklige Platten (9, 10) gebildet, deren Seitenränder ein Sägezahnprofil haben. Im hinteren Stirnteil (3) des Zylinders (1) sind zwei Paare von zueinander rechtwinkligen Nuten (11', 11'', 12', 12'') ausgebildet, einer langen und einer kurzen Nut in jedem Paar (11, 12). Die Länge der kurzen Nuten (11'' und 12'') entspricht der Breite der Platten (9, 10), und die Länge der langen Nuten (11', 12') übersteigt die Breite der Platten (9, 10). In den Wänden der kurzen Nuten (11'', 12'') sind Vertiefungen entsprechend der Form der Zähne des Sägezahnprofils der Platten (9, 10) ausgebildet.

Die Spritze ist zur Verwendung in beliebigen medizinischen Anstalten und für den individuellen Gebrauch bestimmt.

EP 0 425 678 A1

FIG.6

2

# EINMALSPRITZE FÜR INJEKTIONEN

## Technisches Gebiet

Die Erfindung bezieht sich auf die medizinische Technik, und zwar auf Einmalspritzen die für Injektionen verwendet werden.

## Früherer Stand der Technik

Weit bekannt sind Einmalspritzen für Injektionen mit einem Zylinder, einem in diesem angeordneten Kolben mit Kolbenstange, einer Öffnung im hinteren Stirnende des Zylinders zum Durchtritt der Kolbenstange und mit einer Vorrichtung zum Aufsetzen der Nadel am vorderen Stirnende des Zylinders (beispielsweise die Einmalspritzen aus der Produktion der Firma Europe Trumo, Belgien). Diese Spritzen unterscheiden sich konstruktiv praktisch in nichts von den in der medizinischen Praxis weit bekannten Spritzen zur mehrmaligen Verwendung, mit Ausnahme dessen, daß sie aus einem billigeren (polymeren) Werkstoff ausgeführt sind und nicht sterilisiert werden müssen. Dies bedeutet, daß die Konstruktion der bekannten Einmalspritzen die Möglichkeit ihrer mehrmaligen Verwendung erlaubt. Hierzu kann es kommen aus Unachtsamkeit oder mangelnder Gewissenhaftigkeit des medizinischen Personals und beim Setzen von Injektionen durch Personen in einem durch Rauschgift oder Alkohol berauschten Zustand. Die genannten Umstände sind deshalb wichtig, weil in diesen Fällen eine Ansteckung durch den AIDS-Virus, der infektiösen Hepatitis und anderer Krankheiten möglich ist.

Bekannt ist auch eine Einmalspritze mit einem Zylinder, einem in diesem angeordneten Kolben mit Kolbenstange, einer Öffnung im hinteren Stirnende des Zylinders zum Durchtritt der Kolbenstange und einer Vorrichtung zum Aufsetzen der Nadel (EP, A, 0 282 097).

Bei dieser Spritze ist die Nadel an einer Scheibe befestigt,die im vorderen Teil des Zylinders untergebracht ist und sich längs dessen Achse hin und her verschieben kann. Der hinter der Scheibe im Zylinder angeordnete Kolben, der starr mit der Kolbenstange verbunden ist, ist nicht mit der Scheibe verbunden, sondern hat Greifer, die zur Kupplung des Kolbens mit der Scheibe bei deren Zusammenwirken an den Stirnflächen dienen. Bei Beendigung einer Injektion und bei der Berührung der Stirnflächen des Kolbens und der Scheibe wird der Kolben mittels der Greifer starr mit der die Nadel tragende Scheibe gekuppelt, was zur Folge hat, daß bei einem wiederholten Aufziehen des Injektionsmittels in die Spritze die Scheibe mit der Nadel, dem Kolben folgend, ins Innere des Zylinders der Spritze eingezogen wird. Wenn die Nadel ins Innere des Zylinders eintritt, kommt es zu ihrer Versetzung bezüglich der Achse des Zylinders. Ein Versuch einer wiederholten Injektion führt zum Bruch der Injektionsnadel.

Eine solche Konstruktion läßt jedoch die Möglichkeit einer wiederholten Verwendung der Spritze zu, da der Kolben erst in seiner Endlage im vorderen Teil des Zylinders mit der Scheibe gekuppelt wird. Nur dies bewirkt ein Einziehen der Nadel in den Zylinder, sodaß die Spritze zur wiederholten Verwendung untauglich wird. Wenn der Kolben bei der Durchführung einer Injektion nicht bis in seine Endlage vorgeschoben wird, so kann mit der Spritze eine beliebige Anzahl von Injektionen unter Ausnutzung fast des vollständigen Volumens des Zylinders durchgeführt werden.

## Offenbarung der Erfindung

Der Erfindung liegt die Aufgabe zugrunde, eine Einmalspritze für Injektionen zu schaffen, die die Möglichkeit einer wiederholten Verwendung ausschließt.

Die gestellte Aufgabe wird dadurch gelöst, daß bei einer Einmalspritze für Injektionen mit einem Zylinder, einem in diesem angeordneten Kolben mit einer Kolbenstange, einer Öffnung im hinteren Stirnteil des Zylinders zum Durchtritt der Kolbenstange und einem Ansatz zum Aufsetzen der Nadel am vorderen Stirnende des Zylinders erfindungsgemäß die Kolbenstange auf ihrer Länge wenigstens zwei Abschnitte verschiedenen Querschnitts aufweist, nämlich einen ersten, unmittelbar am Kolben angrenzenden Abschnitt, dessen Länge die Dicke des hinteren Stirnteils übersteigt und der die Form eines Stabes hat, dessen Durchmesser kleiner ist als der Durchmesser der Öffnung im hinteren Stirnteil des Zylinders, und einen zweiten Abschnitt, dessen Länge zusammen mit dem ersten Abschnitt und dem Kolben die Länge des Zylinders übersteigt und der im Querschnitt kreuzförmig ausgebildet ist und aus zwei zueinander rechtwinkligen Platten besteht, deren Seitenränder ein Sägezahnprofil haben, wobei die Zähne der Profile einer Platte entgegen den Zähnen der Profile der zweiten Platte geneigt sind, und wobei im hinteren Teil des Zylinders zwei Paare zueinander rechtwinkliger und sich auf der Achse des Zylinders schneidender durchgehender Nuten, eine lange und eine kurze in jedem Paar, ausgebildet sind, welche Paare einander gegenüber bezüglich der Zylinderachse gedreht sind, wobei die Länge der kurzen Nuten der Breite der Platten der kreuzförmigen Kolbenstange entspricht und die Länge der langen Nuten die Breite der Platten der kreuzförmigen Kolbenstange übersteigt, und wenigstens eine der Wände der kurzen Nuten profiliert ausgebildet ist und Vertiefungen entsprechend der Form der Zähne des Sägezahnprofils der Platten hat.

Die Einmalspritze für Injektionen entsprechend der vorliegenden Erfindung schließt die Möglichkeit einer wiederholten Durchführung von Injektionen aus, ist einfach in der Herstellung und zuverlässig im Gebrauch. Die Aufwendungen zur Herstellung der Einmalspritze gemäß der Erfindung übersteigen praktisch nicht die Aufwendungen für die Herstellung der zur Zeit bekannten Einmalspritzen.

Kurzbeschreibung der Zeichnungen

Nachfolgend wird die Erfindung durch die Beschreibung einer konkreten Ausführungsvariante und der beigegebenen Zeichnungen erläutert. Auf diesen zeigt:

Fig. 1 den Zylinder einer Einmalspritze für Injektionen gemäß der Erfindung in isometrischer Darstellung;

Fig. 2 den Kolben mit der Kolbenstange in isometrischer Darstellung;

Fig. 3 das gleiche wie in Fig. 1, Schnitt nach Linie III-III;

Fig. 4 das gleiche wie in Fig. 1, Schnitt nach Linie IV-IV;

Fig. 5 eine Einmalspritze für Injektionen gemäß der Erfindung beim Aufziehen des Injektionsmittels in einer Längsschnittdarstellung;

Fig. 6 eine Einmalspritze für Injektionen gemäß der Erfindung beim Verbringen der Kolbenstange mit dem Kolben aus der Stellung I in die Stellung II in isometrischer Darstellung;

Fig. 7 die Einmalspritze für Injektionen gemäß der Erfindung beim Einführen des Injektionsmittels in einer Längsschnittdarstellung.

Beste Ausführungsform der Erfindung

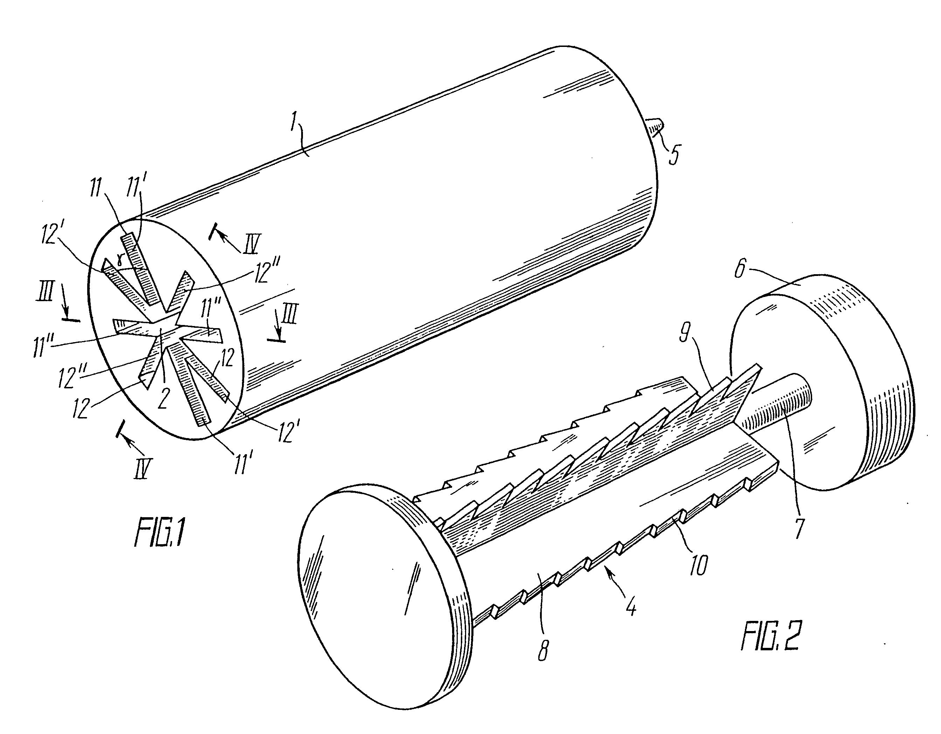

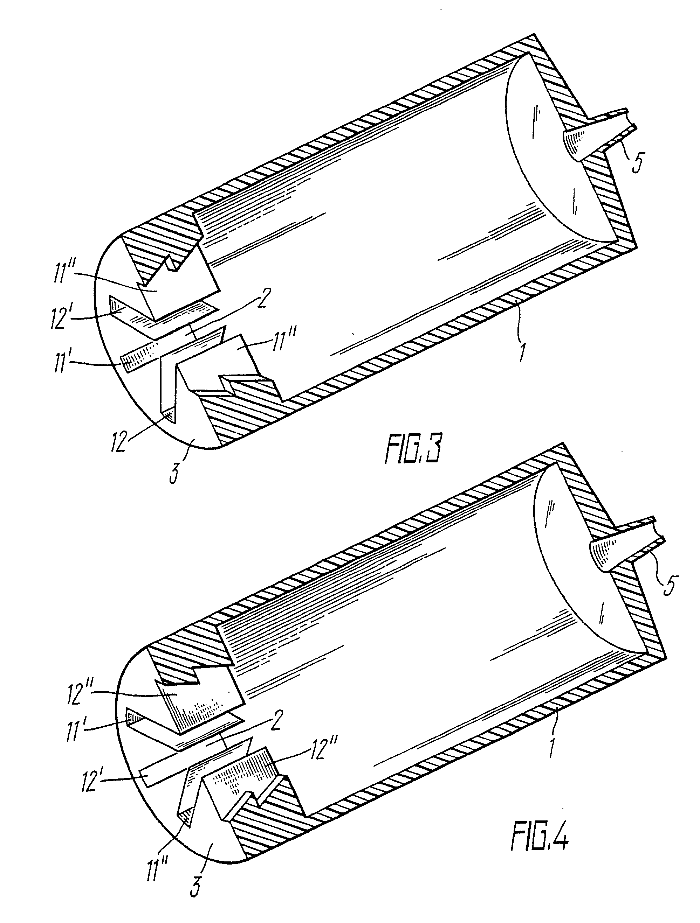

Die Einmalspritze für Injektionen gemäß der Erfindung hat einen Zylinder 1 (Fig. 1, 2, 3, 4) mit einer Zentralöffnung 2 im hinteren Stirnteil 3 zum Durchtritt der Kolbenstange 4 und mit einem Ansatz zum Aufsetzen der Nadel am vorderen Stirnende des Zylinders 1, ausgeführt in Form einer Kanüle 5.

Im Zylinder 1 ist der Kolben 6 mit der Kolbenstange 4 angeordnet (in Fig. 2 ist der Kolben 6 mit der Kolbenstange 4 aus dem Zylinder 1 herausgezogen). Die Kolbenstange 4 hat auf ihrer Länge wenigstens zwei, im beschriebenen Beispiel zwei Abschnitte 7, 8 verschiedenen Querschnitts, wobei der Abschnitt 7, der unmittelbar an den Kolben 6 angrenzt, die Form eines Stabes hat, dessen Durchmesser kleiner ist als der Durchmesser der Zentralöffnung 2 im hinteren Stirnteil 3 des Zylinders 1 und dessen Länge die Dicke der hinteren Stirnwand 3 des Zylinders 1 übersteigt.

Der zweite Abschnitt 8 der Kolbenstange 4 ist im Querschnitt kreuzförmig ausgebildet und von zwei zueinander rechtwinkligen Platten 9 und 10 gebildet, deren Seitenränder ein Sägezahnprofil aufweisen. Die Zähne der Profile der Platte 9 sind in Richtung zum Kolben 6 geneigt und die Zähne der Profile der Platte 10 sind in der entgegengesetzten Richtung geneigt. Die Länge des Abschnitts 8 der Kolbenstange 4 zuzüglich der Länge des Abschnitts 7 der Kolbenstange 4 und der Höhe des Kolbens 6 übersteigt die Länge des Zylinders 1.

Die Öffnung 2 (Fig. 1, 3, 4) im hinteren Teil 3 des Zylinders 1 ist durch zwei Paare 11 und 12 von zueinander rechtwinkligen und sich auf der Achse des Zylinders 1 schneidenden Durchgangsnuten 11', 12' und 11", 12" gebildet.

In jedem Nutenpaar 11 oder 12 gibt es eine lange Nut 11' bzw. 12' und eine kurze Nut 11" bzw. 12". Das Nutenpaar 11 ist bezüglich des Nutenpaares 12 um die Achse des Zylinders 1 um einen Winkel $\propto$ gedreht.

Die Länge der langen Nuten 11' und 12' übersteigt die Breite der Platten 9 oder 10 der kreuzförmigen Kolbenstange und die Länge der kurzen Nuten 11" und 12" entspricht der Breite der Platten 9 und 10 der kreuzförmigen Kolbenstange 4. Wenigstens eine der Wände der kurzen Nuten 11" und 12", im beschriebenen Beispiel beide gegenüberliegenden Wände jeder kurzen Nut 11" und 12" sind profiliertausgebildet, wobei die Wände der Nut 11' profilierte Vertiefungen entsprechend der Form der Zähne des Sägezahnprofils der Platte 9 der kreuzförmigen Kolbenstange 4 und die Wände der Nut 12" profilierte Vertiefungen entsprechend der Form der Zähne des Sägezahnprofils der Platte 10 der kreuzförmigen Kolbenstange 4 aufweisen.

Die Verschiebung der Platte 10 der kreuzförmigen Kolbenstange 4 in der Nut 11" und die Verschiebung der Platte 10 in der Nut 12" ist nur in einer Richtung möglich (und zwar entgegengesetzt für jede der Platten 9, 10), was bedingt ist durch die entsprechende Neigung der Zähne der Profile der Platten 9 und 10 sowie der Form der Vertiefungen der formentsprechenden Zähne in den Wänden der Nuten 11" und 12". Die Verschiebung der Kolbenstange 4 längs der Achse des Zylinders 1 ist nur möglich, wenn sich die Platte 9 in der Nut 11" (Stellung I der Kolbenstange 4) oder wenn sich die Platte 10 in der Nut 12" (Stellung II der Kolbenstange 4) befindet.

Der Gebrauch der Einmalspritze für Injektionen geschieht auf folgende Weise:

In der Ausgangsstellung befindet sich die Kolbenstange 4 so im Zylinder 1 der Spritze, daß der Kolben 6 an der Innenfläche des vorderen Stirnteils des Zylinders 1 mit dem Ansatz 5 anliegt. Dabei befinden sich die Zähne des Sägezahnprofils der Platte 9 der kreuzförmigen Kolbenstange 4 in der kurzen Nut 11".

Die Platte 10 liegt in der langen Nut 11', deren Länge größer ist als die Breite der Platte 10, wodurch eine freie Bewegung der letzteren durch die Nut 11' gewährleistet ist.

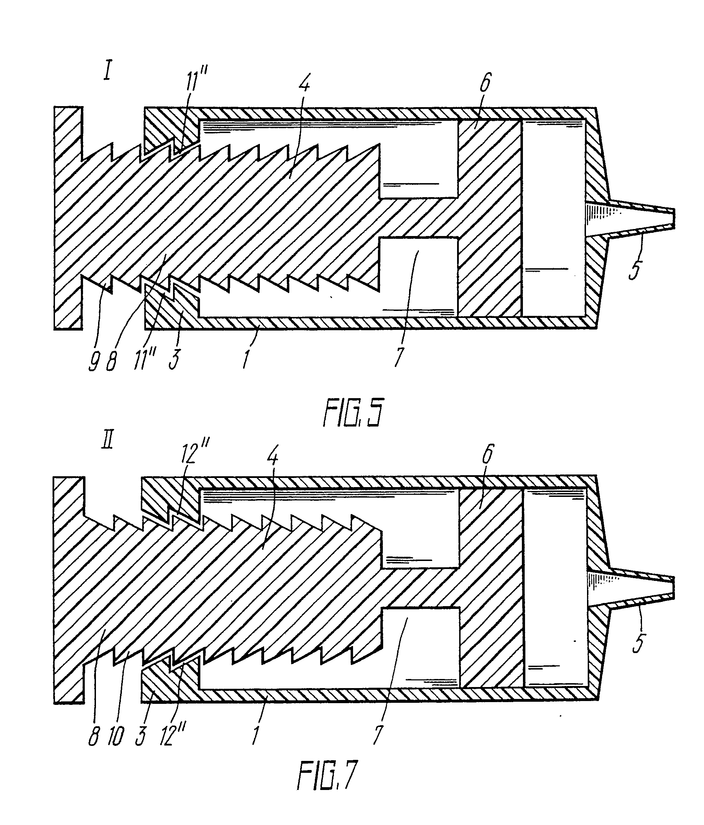

Zum Aufziehen des Mittels in die Spritze wird die Injektionsnadel auf den Ansatz 5 gesetzt und die Kolbenstange 4 mit dem Kolben 6 aus dem Zylinder 1 herausgezogen (Fig. 5). Die Kolbenstange 4 befindet sich dabei in der Stellung I. In diesem Zyklus ist die Bewegung der Kolbenstange 4 mit dem Kolben 6 nur in einer Richtung möglich, was gewährleistet ist durch den Durchgang der Zähne der Profile der Platte 9 durch die Vertiefungen in den Wänden der Nut 11".

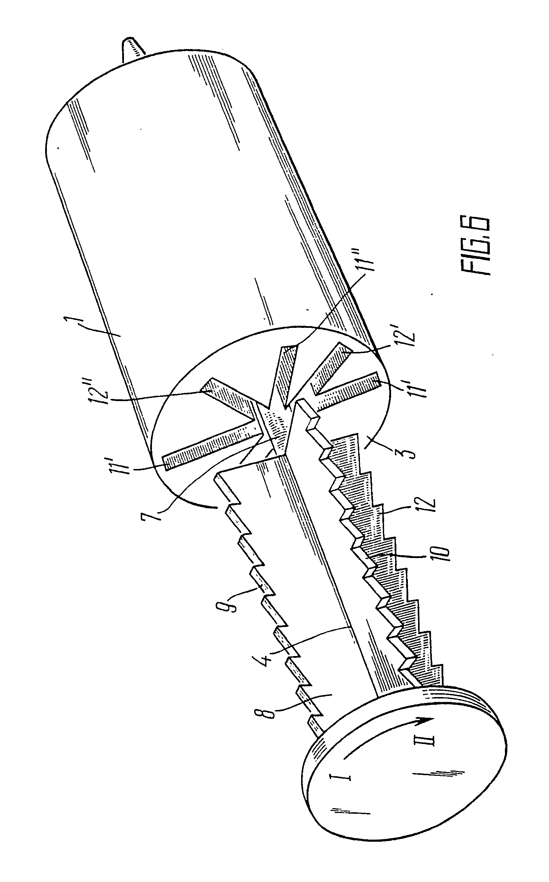

Nach dem vollständigen Austritt des Abschnitts 8 der Kolbenstange 4 aus dem Zylinder 1 ergibt sich die Möglichkeit einer Drehung der Kolbenstange 4 um die Achse des Zylinders 1, weil sich jetzt der Abschnitt 7 der Kolbenstange 4 in der Öffnung 2 des hinteren Stirnendes 3 des Zylinders 1 befindet und der Durchmesser dieses Abschnitts 7 kleiner ist als der Durchmesser des zentralen Teils der Öffnung 2 und seine Länge größer ist als die Dicke des hinteren Stirnteils 3 (Fig. 6). Ein vollständiges Herausziehen der Kolbenstange 4 aus dem Zylinder 1 ist nicht möglich, weil der Durchmesser des Kolbens 6 die Abmessungen der Öffnung 2 im hinteren Stirnteil 3 des Zylinders 1 wesentlich übersteigt.

Zum Einführen des Arzneimittels muß die Platte 10 der kreuzförmigen Kolbenstange gegenüber der Nut 12" im hinteren Stirnteil 3 des Zylinders 1 gestellt werden, d.h. die Kolbenstange 4 durch eine Drehung um die Achse des Zylinders 1 aus der Stellung I in die Stellung II verbracht werden, wobei der Drehwinkel, beispielsweise 45°, gleich dem Winkel ∝ zwischen den Nutenpaaren 11 und 12 (Fig. 6) ist.

Diese Stellung ist unerläßlich, weil eine beliebige andere Stellung der Kolbenstange 4 keine Möglichkeit bietet, den Kolben 6 in den Zylinder 1 der Spritze einzuschieben. Dies wird gewährleistet durch die Form der Sägezahnprofile der Platten 9 und 10 der kreuzförmigen Kolbenstange 4 sowie der Vertiefungen in den Wänden der Nuten 11" und 12". Die Verschiebung der Kolbenstange 4 mit dem Kolben 6 bei dem Zyklus des Einführens des Arzneimittels ist wegen des einseitig gerichteten Durchgangs des Sägezahnprofils der Platte 10 durch die Vertiefungen in den Wänden der Nut 12" nur in einer Richtung möglich.

Da eine Rückbewegung der Kolbenstange 4 nicht möglich ist, wird nach der Einführung des Mittels die Spritze zur weiteren Verwendung untauglich und muß entsorgt werden.

Eine breite Anwendung der vorgeschlagenen Spritze kann praktisch eine Ansteckung des Patienten bei Injektionen durch den AIDS-Virus, der infektiösen Hepatitis und anderer Krankheiten ausschließen, die bei parenteraler Applikation von Arzneimitteln übertragen werden können.

Gewerbliche Anwendbarkeit

Die vorgeschlagene Spritze für Injektionen kann in beliebigen medizinischen Einrichtungen sowie zum privaten Gebrauch verwendet werden.

**Ansprüche**

1. Einmalspritze für Injektionen mit einem Zylinder (1), einem in diesem angeordneten Kolben (6) mit Kolbenstange (4), einer Öffnung (2) im hinteren Stirnteil (3) des Zylinders (1) zum Durchtritt der Kolbenstange (4) und einem Ansatz zum Aufsetzen der Nadel am vorderen Stirnteil des Zylinders (1),
dadurch gekennzeichnet, daß die Kolbenstange (4) auf ihrer Länge wenigstens zwei Abschnitte (7, 8) verschiedenen Querschnitts aufweist, nämlich
einen ersten, unmittelbar am Kolben (6) angrenzenden Abschnitt (7) einer die Dicke des hinteren Stirnteils (3) überschreitenden Länge und in Form eines Stabes, dessen Durchmesser kleiner ist als der Durchmesser der Öffnung (2) im hinteren Stirnteil (3) des Zylinders (1) und einen zweiten Abschnitt (8), dessen Länge zusammen mit dem ersten Abschnitt (7) und dem Kolben (6) die Längs des Zylinders (1) übersteigt, und der im Querschnitt kreuzförmig ausgebildet ist und durch zwei zueinander rechtwinklige Platten (9, 10) gebildet ist, deren Seitenränder ein Sägezahnprofil haben, wobei die Zähne der Profile einer Platte (9) entgegen den Zähnen der Profile der zweiten Platte (10) geneigt sind,
und daß im hinteren Stirnteil (3) des Zylinders (1) zwei Paare (11 und 12) zueinander rechtwinkliger und sich auf der Achse des Zylinders (1) schneidender Nuten (11', 11", 12', 12"), eine lange und eine kurze in jedem Paar, ausgeführt sind, welche Paare zueinander um die Zylinderachse gedreht sind,

wobei die Länge der kurzen Nuten (11" und 12") der Breite der Platten (9, 10) der kreuzförmigen Kolbenstange (4) entspricht und die Länge der langen Nuten (11', 12') die Breite der Platten (9, 10) der kreuzförmigen Kolbenstange (4) übersteigt, und wenigstens eine der Wände der kurzen Nuten (11", 12") profiliert ausgebildet ist und Vertiefungen entsprechend der Form der Zähne des Sägezahnprofils der Platten (9, 10) aufweist.

FIG.1

FIG.2

FIG.3

FIG.4

FIG. 5

FIG. 7

FIG.6

# INTERNATIONAL SEARCH REPORT

International Application No PCT/SU 89/00260

## I. CLASSIFICATION OF SUBJECT MATTER (if several classification symbols apply, indicate all) 6

According to International Patent Classification (IPC) or to both National Classification and IPC

IPC⁵: A 61 M 5/00

## II. FIELDS SEARCHED

| Minimum Documentation Searched 7 | |
|---|---|
| Classification System | Classification Symbols |
| IPC⁴ | A 61 M 5/00, 5/18, 5/24, 5/28, 5/32, 5/315 |

Documentation Searched other than Minimum Documentation
to the Extent that such Documents are Included in the Fields Searched 8

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| A,P | DE, CI, 3811973 (GREIVE MICHAEL) 3 August 1989 see the claims, figure 1 | 1 |
| A | WO, 89/00432 (ASSISTANCE PUBLIQUE et al.) 26 January 1989, the abstract | 1 |
| A | FR, A1, 2613628, (EMERIT ANDRE et al.) 14 October 1988, see the abstract | 1 |
| A | DE, A1, 2458004 (ARZNEIMITTEL GmbH RAVENSBURG APOTHEKER VETTER & CO), 10 June 1976, see the claims, figure 3 | 1 |

* Special categories of cited documents: 16

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 22 February 1990 (22.02.90) | 6 April 1990 (06.04.90) |
| International Searching Authority | Signature of Authorized Officer |
| ISA/SU | |

Form PCT/ISA/210 (second sheet) (January 1985)